# EUROPÄISCHE PATENTSCHRIFT

(11) **EP 1 674 746 B1**
(45) Veröffentlichungstag und Bekanntmachung des Hinweises auf die Patenterteilung: **20.07.2011**
(21) Anmeldenummer: 05026799.6
(22) Anmeldetag: 08.12.2005
(51) Int. Cl.: F16C 27/06, F16C 35/073, A61B 6/03

(54) **Lageranordnung**
Bearing arrangement
Arrangement de palier

(30) Priorität: 23.12.2004 DE 102004062117
(43) Veröffentlichungstag der Anmeldung: 28.06.2006
(73) Patentinhaber: AB SKF, 415 50 Göteborg (SE)
(72) Erfinder: Brandenstein, Manfred, 97776 Eussenheim (DE); Neder, Günter, 97422 Schweinfurt (DE); Neder, Jürgen, 97421 Schweinfurt (DE); Olschewski, Armin, 97422 Schweinfurt (DE); Breunig, Heinz, 63743 Aschaffenburg (DE)
(74) Vertreter: Kohl, Thomas

(56) Entgegenhaltungen:
- DE-A1- 3 425 161
- GB-A- 412 491
- GB-A- 2 269 864
- US-A1- 2004 062 343
- US-B1- 6 471 179
- US-B1- 6 536 953

## Beschreibung

Die Erfindung betrifft eine Lageranordnung für ein medizinisches Gerät, mit der ein rotierendes Bauteil relativ zu einem ortsfesten Gehäuse gelagert wird, insbesondere für einen Computertomographen, wobei die Lageranordnung ein Lager mit einem Innenring und einem Außenring aufweist, wobei der Innenring mit dem zu lagernden Bauteil verbunden ist und wobei der Außenring mit dem Gehäuse verbunden ist und wobei die Verbindung zwischen Innenring und dem zu lagernden Bauteil und/oder zwischen Außenring und Gehäuse durch ein ringförmiges Element hergestellt wird, das aus Kunststoff besteht.

Insbesondere bei Computertomographen besteht die Notwendigkeit, einen trommelförmig ausgebildeten Körper relativ zu einem Gehäuse so zu lagern, dass dieser zwecks Anfertigung von tomographischen Bildern um eine Drehachse gedreht werden kann. In den trommelförmigen Körper ist Dabei zwecks Durchstrahlung des zu untersuchenden Patienten eine Röntgenröhre eingebracht sowie auf der diametral zur Röntgenröhre gegenüberliegenden Seite der Trommel Strahlendetektoren, die die emittierte Röntgenstrahlung aufnehmen.

Eine Lageranordnung der gattungsgemäßen Art, allerdings nicht für medizinische Anwendungen, ist beispielsweise aus der GB 412 491 A, aus der DE 34 25 161 A1 oder aus der GB 2 269 864 A bekannt. Ein anderes Lagerkonzept ist aus der DE-OS 15 75 635 bekannt. Die dort beschriebene Lageranordnung ist für eine elektrische Maschine vorgesehen. Dabei ist der Lageraußenring über ein Dämpfungselement bzw. über mehrere über den Umfang des Lageraußenrings gleichmäßig verteilt angeordnete Dämpfungselemente mit dem Gehäuse bzw. dem Gestell verbunden. Der Lagerinnenring trägt das sich drehende Bauteil.

Aus diversen Schriften, beispielsweise aus der DD-PS 78 523, der DE 25 55 021 C2 und der DE 30 32 820 C2, sind ebenfalls Lager für diverse Anwendungen bekannt, bei denen Elastomerblöcke oder aus elastomerem Material bestehende Hülsen eingesetzt werden, um dem Lager eine verbesserte Dämpfungseigenschaft zu verleihen.

Ein Wälzlager für einen Kernspin-Tomographen mit magnetischen Wälzkörpern und mit einem Innenring und einem Außenring, zwischen denen die Wälzkörper abrollen können, ist aus der WO 02/27203 A1 bekannt. Dort ist weiter vorgesehen, dass der Außenring von einem nichtmagnetischen Ring umgeben ist.

Bekannt sind schließlich für computertomographische Anlagen Lageranordnungen, bei denen Draht-Lager mit eingesetzten Dämpfungselementen zur Anwendung kommt. Die drehbare Trommel wird dort also mittels eines Kugellagers mit Laufdrähten gelagert, wobei die Laufdrähte in dämpfenden Kunststoffeinlagen platziert sind. Dabei kommen dann zumeist Keramikkugeln als Wälzkörper für das Lager zum Einsatz.

Es hat sich herausgestellt, dass die vorbekannten Lagersysteme insbesondere in Computertomographen noch nicht den Anforderungen entsprechen, weil die Lagerung im Betrieb, also bei Drehung der Trommel, eine relativ hohe Geräuschentwicklung aufweist. Die Forderung nach einem geräuscharmen Lager, das höchstens 55 dB(A) erzeugt, kann derzeit kaum erfüllt werden. Die Dämpfung der Lageranordnung reicht also bislang nicht aus.

Ein weiteres Problem, das auch in den Zusammenhang mit der Geräuschentwicklung gebracht wird, besteht darin, dass die rotierende Trommel im Betrieb eine nicht unerhebliche Ovalität aufweist, d. h. das Lager ist bislang nicht in der Lage, die Trommel so zu fassen und zu lagern, dass sie eine weitgehend runde Gestalt beibehält.

Der Erfindung liegt daher die **Aufgabe** zugrunde, eine Lageranordnung der eingangs genannten Art so weiterzubilden, dass insbesondere, aber nicht nur, bei Anwendung in einem Computertomographen ein besonders ruhiger und gleichmäßiger Lauf sichergestellt ist. Die Lageranordnung soll insbesondere bei zu lagernden Teilen, die eine relativ geringe Eigensteifigkeit aufweisen, einen guten Rundlauf sicherstellen und so zu erhöhter Laufruhe beitragen.

Die **Lösung** dieser Aufgabe durch die Erfindung ist dadurch gekennzeichnet, dass die Erstreckung des Innenrings in radiale Richtung zwischen 15 mm und 30 mm beträgt, wobei der Innendurchmesser des Innenrings zwischen 1.000 mm und 2.000 mm liegt.

Bevorzugt ist der Kunststoff ein thermoplastisches Elastomer (TPE). Dabei kann als thermoplastisches Elastomer ein Block-Copolymer vorgesehen werden, wobei insbesondere an ein Styrol-Block-Copolymer, ein thermoplastisches Copolyester-Elastomer, ein thermoplastisches Polyurethan-Elastomer oder ein Polyether-Block-Amid gedacht ist. Alternativ kann als thermoplastische Elastomer auch eine Elastomerlegierung vorgesehen werden, wobei insbesondere ein thermoplastisches Polyolefin mit unvernetzter Elastomerphase oder ein thermoplastisches Vulkanisat mit chemisch vernetztem Elastomeranteil ins Auge gefasst werden kann.

Das vorgesehene Material für das ringförmige Element führt in überraschender Weise bei der spezifischen Anwendung in medizinischen Geräten, insbesondere bei Computertomographen, zu einem sehr guten Dämpfungsverhalten der Lageranordnung, was sich vor allem in der genannten Anwendung dadurch positiv auszeichnet, dass der Lauf des Tomographen geräuscharm ist.

Eine Fortbildung sieht vor, dass lediglich zwischen Innenring und dem zu lagernden Bauteil das ringförmige Element angeordnet ist.

Besonders vorteilhaft ist es, wenn das ringförmige Element an seiner dem Lagerring zugewandten Seite kegelförmig ausgebildet ist, wobei die vom ringförmigen Element kontaktierte Fläche des Lagerrings eine korrespondierende Kegelform aufweist. Der Kegelwinkel des ringförmigen Elements und des Lagerrings liegt dabei bevorzugt zwischen 2° und 8°. Weiterhin können Mittel vorgesehen werden, mit denen der Lagering relativ zu dem ringförmigen Element in axiale Richtung des Lagers verstellbar und festlegbar ist. Mit diesen Mitteln kann die Vorspannung im Lager eingestellt werden.

Das ringförmige Element kann durch einzelne Segmente, insbesondere durch vier Segmente, gebildet werden, die in Umfangsrichtung aneinandergrenzend angeordnet sind. Ferner kann ein axiales Fixieren des ringförmigen Elements dadurch erreicht werden, dass es in einer ringförmig ausgebildeten Nut im zu lagernden Bauteil angeordnet ist..

Eine weitere Verbesserung des Dämpfungsverhaltens kann erreicht werden, wenn gemäß einer bevorzugten Ausgestaltung der Erfindung weiter vorgesehen wird, dass das ringförmige Element in seinem den Lagerring kontaktierenden Bereich mindestens eine Nut aufweist, in der ein O-Ring angeordnet ist.

Eine weitere Maßnahme, um den Rundlauf des zu lagernden Teils zu verbessern, kann darin bestehen, dass sowohl der Innenring als auch der Außenring als einteilige Elemente ausgebildet sind und eine im wesentlichen hohlzylindrische Grundkontur aufweisen, wobei die Erstreckung des Außenrings in radiale Richtung mindestens das Doppelte, vorzugsweise mindestens das Dreifache, der Erstreckung des Innenrings in radiale Richtung beträgt.

Das zu lagernde Bauteil kann trommelförmig ausgebildet sein und eine im Verhältnis zur Steifigkeit des Außenrings des Lagers geringe Eigensteifigkeit aufweisen.

Das Lager ist mit Vorteil als Wälzlager ausgebildet, d. h. zwischen Innenring und Außenring sind Wälzkörper, insbesondere Kugeln, angeordnet. Bevorzugt ist von Haus aus vorgesehen, dass Innenring, Außenring und dazwischen angeordnete Wälzkörper so toleriert sind, dass Vorspannung im Lager vorliegt.

Die genannte Fortbildung stellt also darauf ab, dass die relativ gering eigensteife Trommel des Tomographen von dem dünnwandigen Innenring des Lagers gefasst wird, wobei durch den im Verhältnis steif ausgebildeten Außenring dem Innenring und in der Folge auch der Trommel eine hohe Rundheit verliehen wird, so dass ein geräuscharmer Lauf der Lageranordnung gewährt wird.

Das Vorhandensein einer hinreichenden Vorspannung im Lager begünstigt die Zentrierwirkung, die der relativ massiv ausgebildete Außenring des Lagers über die Wälzkörper und den Innenring auf die relativ wenig eigensteife Trommel ausübt.

Der Außenring kann ferner über mindestens ein Dämpfungselement mit dem Gehäuse verbunden sein, was die Übertragung von störenden Schwingungen erschwert. Dabei kann eine Anzahl Dämpfungselemente vorgesehen werden, die äquidistant über den Umfang des Außenrings verteilt angeordnet sind.

Zur Festlegung des Außenrings am Gehäuse kann vorgesehen werden, dass sich Befestigungsmittel, insbesondere Schrauben, in axiale Richtung durch den Außenring, das Dämpfungselement bzw. die Dämpfungselemente und das Gehäuse erstrecken.

Das Dämpfungselement kann aus Gummi oder aus Elastomermaterial bestehen, insbesondere aus thermoplastischem oder aus duroplastischem Kunststoff. Die Lagerringe können aus nicht-magnetischem Material gefertigt sein.

Mit der erfindungsgemäßen Ausgestaltung wird eine Lageranordnung geschaffen, die sich durch einen besonders ruhigen Lauf auszeichnet. Bei der Anwendung in Computertomographen werden Geräuschentwicklung durch die Lagerung von weniger als 55 dB(A) möglich.

Die zu lagernde Trommel des Tomographen wird durch die vorgeschlagene Lageranordnung gut zentriert gehalten, so dass die Ovalität der Trommel gering ist. Dennoch wird durch die erfindungsgemäße Ausgestaltung eine optimale Dämpfung des Systems erreicht. Die auf das System wirkenden Schwingungen werden ― sowohl in axialer als auch in radialer Richtung ― gut gedämpft.

In der Zeichnung ist ein Ausführungsbeispiel der Erfindung dargestellt. Es zeigen:
- Fig. 1: den Radialschnitt durch die Lageranordnung eines Computertomographen und
- Fig. 2: in perspektivischer Ansicht einen Teil der Lageranordnung mit teilweise geschnittenen Teilen.

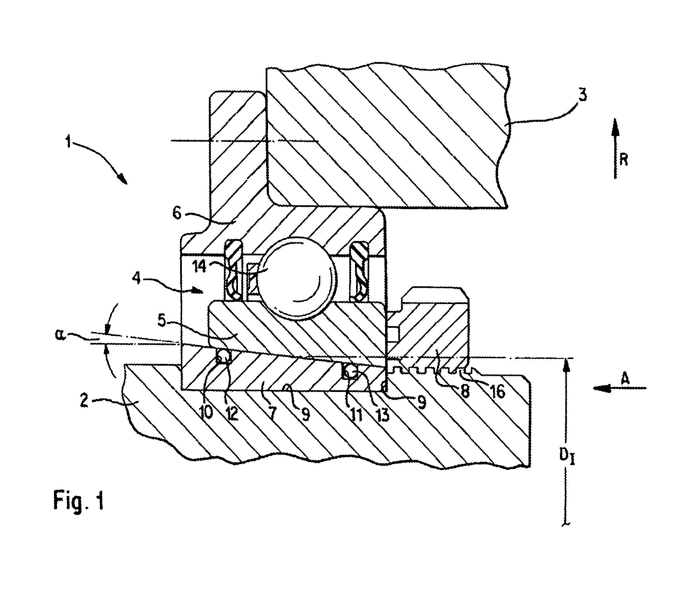

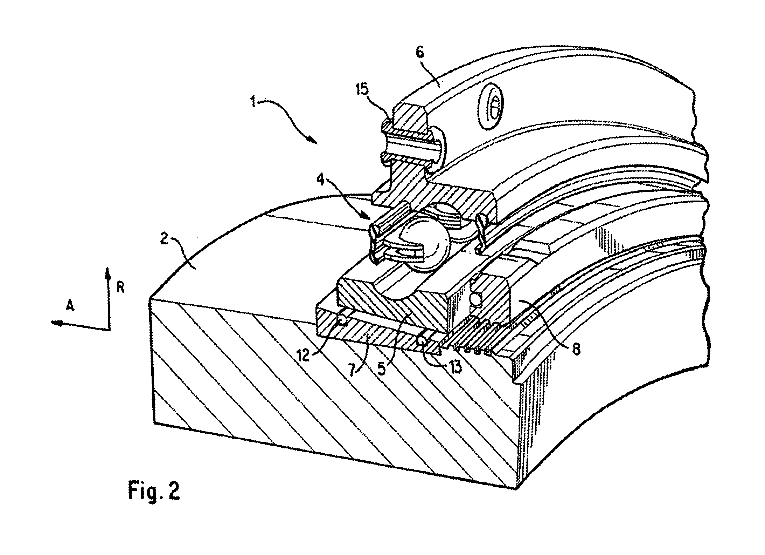

In Figur 1 ist der Schnitt durch die obere Hälfte einer Lageranordnung 1 zu sehen. Figur 2 korrespondiert mit ihrer perspektivischen Darstellung zu Figur 1.

Die Lageranordnung 1 lagert ein rotierendes Bauteil 2 in Form der Trommel eines Computertomographen relativ zu einem ortsfesten Gehäuse 3. Das Bauteil 2 ist dabei aus Gewichtsgründen relativ dünnwandig ausgebildet, so dass es leicht zu einer unerwünschten Ovalität neigt.

Die Lagerung des Bauteils 2 wird durch ein Lager 4 bewerkstelligt, das vorliegend als einreihiges Rillenkugellager ausgebildet ist. Es ist aber genauso möglich, andere Lagertypen einzusetzen. Das Lager 4 weist einen Innenring 5 und einen Außenring 6 auf, zwischen denen in bekannter Weise Wälzkörper 14 in Form von Kugeln angeordnet sind. Je nach Bedarf bestehen die Kugeln 14 entweder klassisch aus Stahl oder aus Keramik.

Wie der Schnittdarstellung von Figur 1 gut entnommen werden kann, sind sowohl der Innenring 5 als auch der Außenring 6 als einteilige Elemente ausgebildet, d. h. sie bestehen aus einem einstückigen Ring. Dabei ist das gesamte Lager 4 als sog. Großlager ausgebildet, d. h. der Außendurchmesser des Außenrings 6 ist größer als ca. 400 mm.

Im Ausführungsbeispiel beträgt die radiale Erstreckung (in radialer Richtung R) des Innenrings 5 ca. 15 mm bis 30 mm, wobei der Innendurchmesser D_{I} des Innenrings 5 zwischen 1.000 mm und 2.000 mm liegt.

Die radial innenliegende Seite des Innenrings 5 ist kegelförmig ausgebildet. Der Kegelwinkel α beträgt etwa 4°. Zwischen Innenring 5 und rotierendem Bauteil 2 ist ein ringförmiges Element 7 angeordnet, das an seiner radial außenliegenden Seite ebenfalls kegelförmig ausgebildet ist, und zwar korrespondierend zum Kegelwinkel des Innenrings 5.

Das ringförmige Element 7 ist dabei in eine Nut 9 im Bauteil 2 eingesetzt. Damit dies einfach zu bewerkstelligen ist und um auch ansonsten die Montage des ringförmigen Elements 7 zu erleichtern, besteht es aus mehreren Segmenten, die aneinandergrenzend über den Umfang des Bauteils 2 zusammengesetzt sind.

Mittel 8 in Form einer auf ein Gewinde 16 aufgeschraubten Wellenmutter erlauben die Einstellung und Fixierung des Innenrings 5 in axialer Richtung A relativ zum ringförmigen Element 7, wodurch die Vorspannkraft der gesamten Lageranordnung beeinflusst werden kann.

In das ringförmige Element 7 sind im Ausführungsbeispiel zwei Nuten 10 und 11 axial versetzt eingearbeitet, in die jeweils in Gummi-O-Ring 12 und 13 eingesetzt ist.

Die Befestigung des Lageraußenrings 6 am Gehäuse 3 erfolgt ― wie es in Fig. 2 erkennbar ist ― mittels Dämpfungselementen 15, die vorliegend aus Gummi- oder Kunststoffhülsen bestehen, die in Bohrungen im Außenring 6 eingebracht sind. In die Bohrungen und durch die Gummi- oder Kunststoffhülsen werden Befestigungsschrauben zum Anschrauben des Lagerrings 6 ans Gehäuse 3 gesteckt.

Eine besondere Bedeutung hat die Wahl des Materials des ringförmigen Elements 7. Als Kunststoff kommt hier ein thermoplastisches Elastomer zum Einsatz.

Thermoplastische Elastomere (TPE) liegen mit ihren Eigenschaft zwischen thermoplastischen Kunststoffen und Elastomeren (Gummimaterialien), wobei die jeweils positiven Eigenschaften beider Materialgruppen vereinigt werden. Zum einen können relativ hohe mechanische Steifigkeiten erreicht werden; andererseits ist das Dämpfungsverhalten des Materials sehr gut.

Wie Thermoplaste werden auch thermoplastische Elastomere bei Wärmzufuhr plastisch und erstarren wieder bei Abkühlung. Im Gegensatz zur chemischen Vernetzung bei den Elastomeren handelt es sich bei den thermoplastischen Elastomeren um eine physikalische Vernetzung, die durch erneuten Wärmeeintrag auch wieder reversibel ist.

Gemäß einer Ausgestaltung der Erfindung werden als thermoplastische Elastomere Block-Copolymere eingesetzt, die aus Blöcken verschiedener Härtestufen gebildet werden. Vertreter sind hier Styrol-Block-Copolymere (TPE-S), thermoplastische Copolyester-Elastomere (TPE-E), thermoplastische Polyurethan-Elastomere (TPE-U) oder Polyether-Block-Amide (TPE-A).

Es können auch Elastomerlegierungen eingesetzt werden, die aus zwei-phasigen Systemen bestehen, bei denen vernetzte oder unvernetzte Elastomerpartikel in einer Thermoplastmatrix vermischt sind. Vertreter sind hier thermoplastische Polyolefine mit unvernetzter Elastomerphase (TPE-O) und thermoplastische Vulkanisate mit chemisch vernetztem Elastomeranteil (TPE-V).

Um ein Optimum an Geräuschreduzierung zu erreichen, können die WälzkörperLaufbahnen in Außen- und Innenring nach dem Schleifen gehont oder rolliert werden. D. h. nach dem Schleifen wird eine weitere Feinbearbeitung vorgesehen, um bestmögliche Resultate zu erzielen.

### Bezugszeichenliste

- 1: Lageranordnung
- 2: rotierendes Bauteil
- 3: ortsfestes Gehäuse
- 4: Lager
- 5: Innenring
- 6: Außenring
- 7: ringförmiges Element
- 8: Mittel zum Verstellen und Festlegen (Mutter)
- 9: Nut im Bauteil
- 10: Nut im ringförmigen Element
- 11: Nut im ringförmigen Element
- 12: O-Ring
- 13: O-Ring
- 14: Wälzkörper (Kugeln)
- 15: Dämpfungselement
- 16: Gewinde

- R: radiale Richtung
- A: axiale Richtung
- D₁: Innendurchmesser
- α: Kegelwinkel

## Patentansprüche

1. Lageranordnung (1) für ein medizinisches Gerät, mit der ein rotierendes Bauteil (2) relativ zu einem ortsfesten Gehäuse (3) gelagert wird, insbesondere für einen Computertomographen, wobei die Lageranordnung (1) ein Lager (4) mit einem Innenring (5) und einem Außenring (6) aufweist, wobei der Innenring (5) mit dem zu lagernden Bauteil (2) verbunden ist, wobei der Außenring (6) mit dem Gehäuse (3) verbunden ist und wobei die Verbindung zwischen Innenring (5) und dem zu lagernden Bauteil (2) und/oder zwischen Außenring (6) und Gehäuse (3) durch ein ringförmiges Element (7) hergestellt wird, das aus Kunststoff besteht,
**dadurch gekennzeichnet,**
**dass** die Erstreckung des Innenrings (5) in radiale Richtung (R) zwischen 15 mm und 30 mm beträgt, wobei der Innendurchmesser (D₁) des Innenrings (5) zwischen 1.000 mm und 2.000 mm liegt.

2. Lageranordnung nach Anspruch 1, **dadurch gekennzeichnet, dass** der Kunststoff ein thermoplastisches Elastomer ist.

3. Lageranordnung nach Anspruch 2, **dadurch gekennzeichnet, dass** das thermoplastische Elastomer ein Block-Copolymer ist.

4. Lageranordnung nach Anspruch 3, **dadurch gekennzeichnet, dass** das thermoplastische Elastomer ein Styrol-Block-Copolymer, ein thermoplastisches Copolyester-Elastomer, ein thermoplastisches Polyurethan-Elastomer oder ein Polyether-Block-Amid ist.

5. Lageranordnung nach Anspruch 2, **dadurch gekennzeichnet, dass** das thermoplastische Elastomer eine Elastomerlegierung ist.

6. Lageranordnung nach Anspruch 5, **dadurch gekennzeichnet**, das das thermoplastische Elastomer ein thermoplastisches Polyolefin mit unvernetzter Elastomerphase oder ein thermoplastisches Vulkanisat mit chemisch vernetztem Elastomeranteil ist.

7. Lageranordnung nach einem der Ansprüche 1 bis 6, **dadurch gekennzeichnet, dass** lediglich zwischen Innenring (5) und dem zu lagernden Bauteil (2) das ringförmige Element (7) angeordnet ist.

8. Lageranordnung nach einem der Ansprüche 1 bis 7, **dadurch gekennzeichnet, dass** das ringförmige Element (7) an seiner dem Lagerring (5, 6) zugewandten Seite kegelförmig ausgebildet ist, wobei die vom ringförmigen Element (7) kontaktierte Fläche des Lagerrings (5, 6) eine korrespondierende Kegelform aufweist.

9. Lageranordnung nach Anspruch 8, **dadurch gekennzeichnet, dass** der Kegelwinkel (α) des ringförmigen Elements (7) und des Lagerrings (5, 6) zwischen 2° und 8° liegt.

10. Lageranordnung nach Anspruch 8 oder 9, **gekennzeichnet durch** Mittel (8), mit denen der Lagering (5, 6) relativ zu dem ringförmigen Element (7) in axiale Richtung (A) des Lagers (4) verstellbar und festlegbar ist.

11. Lageranordnung nach einem der Ansprüche 1 bis 10, **dadurch gekennzeichnet, dass** das ringförmige Element (7) durch einzelne Segmente, insbesondere durch vier Segmente, gebildet wird, die in Umfangsrichtung aneinandergrenzend angeordnet sind.

12. Lageranordnung nach einem der Ansprüche 1 bis 11, **dadurch gekennzeichnet, dass** das ringförmige Element (7) in einer ringförmig ausgebildeten Nut (9) im zu lagernden Bauteil (2) angeordnet ist.

13. Lageranordnung nach einem der Ansprüche 1 bis 12, **dadurch gekennzeichnet, dass** das ringförmige Element (7) in seinem den Lagerring (5, 6) kontaktierenden Bereich mindestens eine Nut (10, 11) aufweist, in der ein O-Ring (12, 13) angeordnet ist.

14. Lageranordnung nach einem der Ansprüche 1 bis 13, **dadurch gekennzeichnet, dass** sowohl der Innenring (5) als auch der Außenring (6) als einteilige Elemente ausgebildet sind und eine im wesentlichen hohlzylindrische Grundkontur aufweisen, wobei die Erstreckung des Außenrings (6) in radiale Richtung (R) mindestens das Doppelte, vorzugsweise mindestens das Dreifache, der Erstreckung des Innenrings (5) in radiale Richtung (R) beträgt.

15. Lageranordnung nach einem der Ansprüche 1 bis 14, **dadurch gekennzeichnet, dass** das zu lagernde Bauteil (2) trommelförmig ausgebildet ist und eine im Verhältnis zur Steifigkeit des Außenrings (6) des Lagers (4) geringe Eigensteifigkeit aufweist.

16. Lageranordnung nach einem der Ansprüche 1 bis 15, **dadurch gekennzeichnet, dass** das Lager (4) als Wälzlager ausgebildet ist und zwischen Innenring (5) und Außenring (6) Wälzkörper (14), insbesondere Kugeln, angeordnet sind.

17. Lageranordnung nach Anspruch 16, **dadurch gekennzeichnet, dass** Innenring (5), Außenring (6) und dazwischen angeordnete Wälzkörper (14) so toleriert sind, dass Vorspannung im Lager (4) vorliegt.

18. Lageranordnung nach einem der Ansprüche 1 bis 17, **dadurch gekennzeichnet, dass** der Außenring (6) über mindestens ein Dämpfungselement (15) mit dem Gehäuse (3) verbunden ist.

19. Lageranordnung nach Anspruch 18, **gekennzeichnet durch** eine Anzahl Dämpfungselemente (15), die äquidistant über den Umfang des Außenrings (6) verteilt angeordnet sind.

20. Lageranordnung nach Anspruch 18 oder 19, **dadurch gekennzeichnet, dass** das Dämpfungselement (15) aus Gummi oder aus Elastomermaterial, insbesondere aus thermoplastischem oder aus duroplastischem Kunststoff, besteht.

21. Lageranordnung nach einem der Ansprüche 1 bis 20, **dadurch gekennzeichnet, dass** die Lagerringe (5, 6) aus nicht-magnetischem Material bestehen.

## Claims

1. Bearing arrangement (1) for a medical instrument, by means of which a rotating component (2) is borne relative to a fixed housing (3), more particularly for a computed tomography scanner, the bearing arrangement (1) having a bearing (4) with an inner race (5) and an outer race (6), the inner race (5) being connected to the component (2) to be borne, the outer race (6) being connected to the housing (3) and the connection between inner race (5) and the component (2) to be borne and/or between outer race (6) and housing (3) being established by an annular element (7), which consists of plastic,
**characterized**
**in that** the extent of the inner race (5) in the radial direction (R) is between 15 mm and 30 mm, the inner diameter (D_{I}) of the inner race (5) lying between 1000 mm and 2000 mm.

2. Bearing arrangement according to Claim 1, **characterized in that** the plastic is a thermoplastic elastomer.

3. Bearing arrangement according to Claim 2, **characterized in that** the thermoplastic elastomer is a block copolymer.

4. Bearing arrangement according to Claim 3, **characterized in that** the thermoplastic elastomer is a styrene block copolymer, a thermoplastic copolyester elastomer, a thermoplastic polyurethane elastomer or a polyether block amide.

5. Bearing arrangement according to Claim 2, **characterized in that** the thermoplastic elastomer is an elastomer alloy.

6. Bearing arrangement according to Claim 5, **characterized in that** the thermoplastic elastomer is a thermoplastic polyolefin with an uncrosslinked elastomer phase or a thermoplastic vulcanizate with a chemically crosslinked elastomer component.

7. Bearing arrangement according to one of Claims 1 to 6, **characterized in that** the annular element (7) is merely arranged between inner race (5) and the component (2) to be borne.

8. Bearing arrangement according to one of Claims 1 to 7, **characterized in that** the annular element (7) has a conical design on the side thereof facing the bearing race (5, 6), with the area of the bearing race (5, 6) contacted by the annular element (7) having a corresponding conical shape.

9. Bearing arrangement according to Claim 8, **characterized in that** the cone angle (α) of the annular element (7) and the bearing race (5, 6) lies between 2° and 8°.

10. Bearing arrangement according to Claim 8 or 9, **characterized by** means (8) that can be used to adjust and fix the bearing race (5, 6) in the axial direction (A) of the bearing (4) relative to the annular element (7).

11. Bearing arrangement according to one of Claims 1 to 10, **characterized in that** the annular element (7) is formed by individual segments, more particularly by four segments, that are arranged adjacent to one another in the circumferential direction.

12. Bearing arrangement according to one of Claims 1 to 11, **characterized in that** the annular element (7) is arranged in a groove (9) with an annular design that is situated in the component (2) to be borne.

13. Bearing arrangement according to one of Claims 1 to 12, **characterized in that** the annular element (7) has at least one groove (10, 11) in the region thereof that contacts the bearing race (5, 6), with an 0-ring (12, 13) arranged in said groove.

14. Bearing arrangement according to one of Claims 1 to 13, **characterized in that** both the inner race (5) and the outer race (6) are designed as integral elements and have a substantially hollow-cylindrical basic contour, the extent of the outer race (6) in the radial direction (R) being at least two times, preferably at least three times, the extent of the inner race (5) in the radial direction (R).

15. Bearing arrangement according to one of Claims 1 to 14, **characterized in that** the component (2) to be borne has a drum-like design and has low inherent stiffness compared to the stiffness of the outer race (6) of the bearing (4).

16. Bearing arrangement according to one of Claims 1 to 15, **characterized in that** the bearing (4) is designed as a rolling-element bearing and roll bodies (14), more particularly spheres, are arranged between inner race (5) and outer race (6).

17. Bearing arrangement according to Claim 16, **characterized in that** inner race (5), outer race (6) and roll bodies (14) arranged therebetween have such tolerances that there is pretension in the bearing (4).

18. Bearing arrangement according to one of Claims 1 to 17, **characterized in that** the outer race (6) is connected to the housing (3) via at least one damping element (15).

19. Bearing arrangement according to Claim 18, **characterized by** a number of damping elements (15) that are arranged distributed equidistantly over the circumference of the outer race (6).

20. Bearing arrangement according to Claim 18 or 19, **characterized in that** the damping element (15) consists of rubber or of elastomeric material, more particularly of thermoplastic or thermosetting plastic.

21. Bearing arrangement according to one of Claims 1 to 20, **characterized in that** the bearing races (5, 6) consist of nonmagnetic material.

## Revendications

1. Agencement de palier (1) pour un appareil médical, avec lequel un composant rotatif (2) est supporté par rapport à un boîtier fixe (3), en particulier pour un tomographe informatique, l'agencement de palier (1) présentant un palier (4) avec une bague interne (5) et une bague externe (6), la bague interne (5) étant connectée au composant (2) à supporter, la bague externe (6) étant connectée au boîtier (3) et la connexion entre la bague interne (5) et le composant (2) à supporter et/ou entre la bague externe (6) et le boîtier (3) étant créée par un élément de forme annulaire (7), qui se compose de plastique,
**caractérisé en ce que**
l'étendue de la bague interne (5) dans la direction radiale (R) est comprise entre 15 mm et 30 mm, le diamètre intérieur (D_{I}) de la bague interne (5) étant compris entre 1.000 mm et 2.000 mm.

2. Agencement de palier selon la revendication 1, **caractérisé en ce que** le plastique est un élastomère thermoplastique.

3. Agencement de palier selon la revendication 2, **caractérisé en ce que** l'élastomère thermoplastique est un copolymère bloc.

4. Agencement de palier selon la revendication 3, **caractérisé en ce que** l'élastomère thermoplastique est un copolymère bloc de styrène, un copolyester élastomère thermoplastique, un élastomère de polyuréthane thermoplastique, ou un polyéther-bloc-amide.

5. Agencement de palier selon la revendication 2, **caractérisé en ce que** l'élastomère thermoplastique est un alliage d'élastomère.

6. Agencement de palier selon la revendication 5, **caractérisé en ce que** l'élastomère thermoplastique est une polyoléfine thermoplastique à phase élastomère non réticulée ou un vulcanisat thermoplastique à proportion élastomère réticulée chimiquement.

7. Agencement de palier selon l'une quelconque des revendications 1 à 6, **caractérisé en ce que** l'élément de forme annulaire (7) est disposé uniquement entre la bague interne (5) et le composant à supporter (2).

8. Agencement de palier selon l'une quelconque des revendications 1 à 7, **caractérisé en ce que** l'élément de forme annulaire (7) est réalisé en forme de cône sur son côté tourné vers la bague de palier (5, 6), la surface de la bague de palier (5, 6) en contact avec l'élément de forme annulaire (7) présentant une forme conique correspondante.

9. Agencement de palier selon la revendication 8, **caractérisé en ce que** l'angle de conicité (α) de l'élément de forme annulaire (7) et de la bague de palier (5, 6) est compris entre 2° et 8°.

10. Agencement de palier selon la revendication 8 ou 9, **caractérisé par** des moyens (8) avec lesquels la bague de palier (5, 6) peut être déplacée et fixée par rapport à l'élément de forme annulaire (7) dans la direction axiale (A) du palier (4).

11. Agencement de palier selon l'une quelconque des revendications 1 à 10, **caractérisé en ce que** l'élément de forme annulaire (7) est formé par des segments individuels, notamment par quatre segments, qui sont disposés de manière juxtaposée dans la direction périphérique.

12. Agencement de palier selon l'une quelconque des revendications 1 à 11, **caractérisé en ce que** l'élément de forme annulaire (7) est disposé dans une rainure (9) réalisée avec une forme annulaire, dans le composant à supporter (2).

13. Agencement de palier selon l'une quelconque des revendications 1 à 12, **caractérisé en ce que** l'élément de forme annulaire (7) présente dans sa région en contact avec la bague de palier (5, 6) au moins une rainure (10, 11) dans laquelle est disposé un joint torique (12, 13).

14. Agencement de palier selon l'une quelconque des revendications 1 à 13, **caractérisé en ce que** la bague interne (5) ainsi que la bague externe (6) sont réalisées sous forme d'éléments d'une seule pièce et présentent un contour de base essentiellement cylindrique creux, l'étendue de la bague externe (6) dans la direction radiale (R) valant au moins le double, de préférence au moins le triple, de l'étendue de la bague interne (5) dans la direction radiale (R).

15. Agencement de palier selon l'une quelconque des revendications 1 à 14, **caractérisé en ce que** le composant à supporter (2) est réalisé en forme de tambour et présente une faible rigidité propre par rapport à la rigidité de la bague externe (6) du palier (4).

16. Agencement de palier selon l'une quelconque des revendications 1 à 15, **caractérisé en ce que** le palier (4) est réalisé sous forme de palier à roulement et des corps de roulement (14), notamment des billes, sont disposés entre la bague interne (5) et la bague externe (6).

17. Agencement de palier selon la revendication 16, **caractérisé en ce que** la bague interne (5), la bague externe (6) et les corps de roulement (14) disposés entre elles ont des tolérances telles qu'il existe une précontrainte dans le palier (4).

18. Agencement de palier selon l'une quelconque des revendications 1 à 17, **caractérisé en ce que** la bague externe (6) est connectée au boîtier (3) par le biais d'au moins un élément d'amortissement (15).

19. Agencement de palier selon la revendication 18, **caractérisé par** une pluralité d'éléments d'amortissement (15), qui sont répartis de manière équidistante sur la périphérie de la bague externe (6).

20. Agencement de palier selon la revendication 18 ou 19, **caractérisé en ce que** l'élément d'amortissement (15) se compose de caoutchouc ou d'un matériau élastomère, notamment de plastique thermoplastique ou duroplastique.

21. Agencement de palier selon l'une quelconque des revendications 1 à 20, **caractérisé en ce que** les bagues de palier (5, 6) se composent de matériau non magnétique.
